# EUROPÄISCHE PATENTSCHRIFT

(11) **EP 3 429 694 B1**
(45) Veröffentlichungstag und Bekanntmachung des Hinweises auf die Patenterteilung: **13.01.2021**
(21) Anmeldenummer: 17705784.1
(22) Anmeldetag: 31.01.2017
(51) Int. Cl.: A61Q 19/02, A61K 8/97

(54) **HAUTAUFHELLENDES KOSMETIKUM**
SKIN-BRIGHTENING COSMETIC
PRODUIT COSMÉTIQUE A EFFET ÉCLAIRCISSANT

(30) Priorität: 18.03.2016 DE 102016105029
(43) Veröffentlichungstag der Anmeldung: 23.01.2019
(73) Patentinhaber: Ocean Research & Development GmbH, 21465 Reinbek (DE)
(72) Erfinder: KEIMES, Jörg, 21465 Reinbek (DE); GÜNTHER, Patrick, 21521 Aumühle (DE)
(74) Vertreter: Lobemeier, Martin Landolf
(86) Internationale Anmeldenummer: PCT/DE2017/100059
(87) Internationale Veröffentlichungsnummer: WO 2017/157366

(56) Entgegenhaltungen:
- DATABASE GNPD [Online] MINTEL; Februar 2016 (2016-02), "The Lifting Eye Serum", XP002768945, Database accession no. 3745411
- JEFFREY D. LASKIN ET AL: "Identification of a distinct phase during melanogenesis that is sensitive to extracellular pH and ionic strength", JOURNAL OF CELLULAR PHYSIOLOGY, Bd. 103, Nr. 3, 1. Juni 1980 (1980-06-01), Seiten 467-474, XP055361928, US ISSN: 0021-9541, DOI: 10.1002/jcp.1041030312
- YUH-SHUEN CHEN ET AL: "Tyrosinase Inhibitory Effect and Antioxidative Activities of Fermented and Ethanol Extracts of Rhodiola rosea and Lonicera japonica", THE SCIENTIFIC WORLD JOURNAL, Bd. 2013, 1. Januar 2013 (2013-01-01), Seiten 1-5, XP055361971, DOI: 10.1155/2013/612739 in der Anmeldung erwähnt
- Barbara Broakway: "Marine derived ingredients for personal care", IMCD, UK, 1. April 2012 (2012-04-01), XP55362103, Gefunden im Internet: URL:https://www.imcdgroup.com/sites/defaul t/files/IMCD in a Personal Care Magazine article.pdf [gefunden am 2017-04-05]

## Beschreibung

Die Erfindung betrifft ein hautaufhellendes Kosmetikum mit einem die Melanogenese hemmendem Wirkstoff.

Eine besonders helle Haut wird seit langem in unterschiedlichen Kulturen als Schönheitsideal verstanden, sodass hautaufhellende Kosmetika insbesondere gegen Pigmentflecke, wie z.B. Altersflecken oder Sommersprossen, Leberflecke oder einen ungleichmäßigen Teint eingesetzt werden.

Eine Hautaufhellung folgt ästhetischen Gesichtspunkten, die u.a. die soziale Stellung dokumentieren oder zur Verbesserung der wirtschaftlichen Position beitragen sollen, und auch medizinische Indikationen, die Entstellungen der Haut weniger sichtbar machen.

Neben abdeckenden Cremes kommen bleichende Mittel zum Einsatz. Letztere weisen jedoch häufig gefährliche Inhaltsstoffe, z. B. Quecksilber auf, die langfristig zu schwerwiegenden Erkrankungen führen und keine verantwortungsvolle Alternative zu abdeckenden Cremes bieten.

Hautverträgliche hautaufhellende Kosmetika hingegen beinhalten regelmäßig pflanzliche Extrakte, die in die Melaninsynthese eingreifen und die Pigmentproduktion gezielt beeinflussen.

Beispielsweise ist ein pflanzlicher Extrakt (CAS Nr. 97404-52-9) aus Rosenwurz (Rhodiola Rosea) bekannt, der eine Tyrosinasehemmende Wirkung aufweist, die zu einer verminderten Melaninbildung und damit zu einer Hautaufhellung führt (Hindawi Publishing Corporation - The ScientificWorld Journal. Volume 2013, Article-ID 612739, http://dx.doi.org/10.1155/2013/612739).

Die Verwendung von in die Melanogenese eingreifenden Mitteln ist allerdings aufgrund der unterdrückten Melaninbildung) dem damit einhergehenden verringerten Schutz vor UV-Strahlung und dem damit verbundenen grundsätzlich erhöhten Risiko einer sonneninduzierten Hautkrebserkrankung problematisch.

Aufgabe der Erfindung ist es daher, ein hautaufhellendes Kosmetikum zu schaffen, dass der Schädigung der Haut durch Sonneneinstrahlung entgegenwirkt.

Grundgedanke der Erfindung ist es, den negativen Auswirkungen einer aus kosmetischen Gründen vorgenommenen oder medizinisch indizierten Hautaufhellung durch die zellprotektive und zellgenerierende Wirkung des Kosmetikums nach der Erfindung entgegenzuwirken.

Erfindungsgemäß wird also ein hautaufhellendes Kosmetikum mit einem Extrakt aus Rosenwurz (*Rhodiola rosea*) als ein die Melanogenese hemmender Wirkstoff bereitgestellt, das zusätzlich einen Extrakt aus dem Knotentang (*Ascophyllum nodosum*) und einen Extrakt aus der Purpur-Rotalge (*Porphyridium cruentum*) enthält.

Bei dem Extrakt aus dem Knotentang (*Ascophyllum nodosum*) handelt es sich insbesondere um CAS-Nr. 84775-78-0.

Bei dem Extrakt aus der Purpur-Rotalge (*Porphyridium cruentum*) handelt es sich insbesondere um CAS-Nr. 223751-77-7.

Bei dem Extrakt aus Rosenwurz (*Rhodiola rosea)* handelt es sich bevorzugt um CAS-Nr-97404-52-9.

Die sich in dem Kosmetikum vorteilhaft ergänzenden Effekte werden auf die Wirkungen der im Knotentang (*Ascophyllum nodosum)* enthaltenen Polyphenole, der in der Purpur-Rotalge (*Porphyridium cruentum*) enthaltenen Polysaccharide und der in der Rosenwurz (*Rhodiola rosea*) enthaltenden phenolischen Glycoside zurückgeführt.

Ein besonders vorteilhaftes Kosmetikum wird erhalten bei einem Gewichtsverhältnis der Extrakte aus Rosenwurz : Knotentang : Purpur-Rotalge von 2:2:1.

Die die pflanzlichen Extrakte aufweisende Kosmetikum weist eine die Vitalität von Fibroblasten stimulierende Wirkung auf.

Insbesondere weist das die pflanzlichen Extrakte aufweisende Kosmetikum eine die Kollagensynthese steigernde Wirkung auf.

Weiter weist das die pflanzlichen Extrakte aufweisende Kosmetikum eine zellprotektive Wirkung vor dem schädigenden Einfluss von UV-Strahlung auf.

Schließlich weist das die pflanzlichen Extrakte aufweisende Kosmetikum eine zellgenerative Wirkung nach UV-Bestrahlung auf.

Das Kosmetikum wird bevorzugt als eine die Extrakte aufweisende wässrige Lösung bereitgestellt.

Die Erfindung ist für die Verwendung eines hautaufhellenden Kosmetikums mit einem Extrakt aus Rosenwurz (*Rhodiola rosea*) als ein die Melanogenese hemmender Wirkstoff, einem Extrakt aus dem Knotentang (*Ascophyllum nodosum*) und einen Extrakt aus der Purpur-Rotalge (*Porphyridium cruentum*), mit zellprotektiver und zellgenerativer Wirkung bei (gegebenenfalls medizinisch indizierten) kosmetischen Behandlungen zur Entfernung von Pigmentierungen gedacht. Dabei kann das Kosmetikum wie vorstehend erläutert vorteilhaft weitergebildet sein.

Die Erfindung richtet sich damit insbesondere an die Hersteller von kosmetischen Formulierungen zum Herstellen eines hautaufhellenden Kosmetikums mit einem Extrakt aus Rosenwurz (*Rhodiola rosea*) als ein die Melanogenese hemmender Wirkstoff, einem Extrakt aus dem Knotentang (*Ascophyllum nodosum)* und einen Extrakt aus der Purpur-Rotalge (*Porphyridium cruentum*), mit zellprotektiver und zellgenerativer Wirkung bei (gegebenenfalls medizinisch indizierten) kosmetischen Behandlungen zur Entfernung von Pigmentierungen. Dabei kann das herzustellende Kosmetikum und damit das Verfahren zum Herstellen des Kosmetikums wie vorstehend erläutert vorteilhaft weitergebildet sein.

Die Erfindung wird anhand eines besonders bevorzugt ausgestalteten Ausführungsbeispiels und anhand der beigefügten Zeichnungen näher erläutert. Es zeigen:

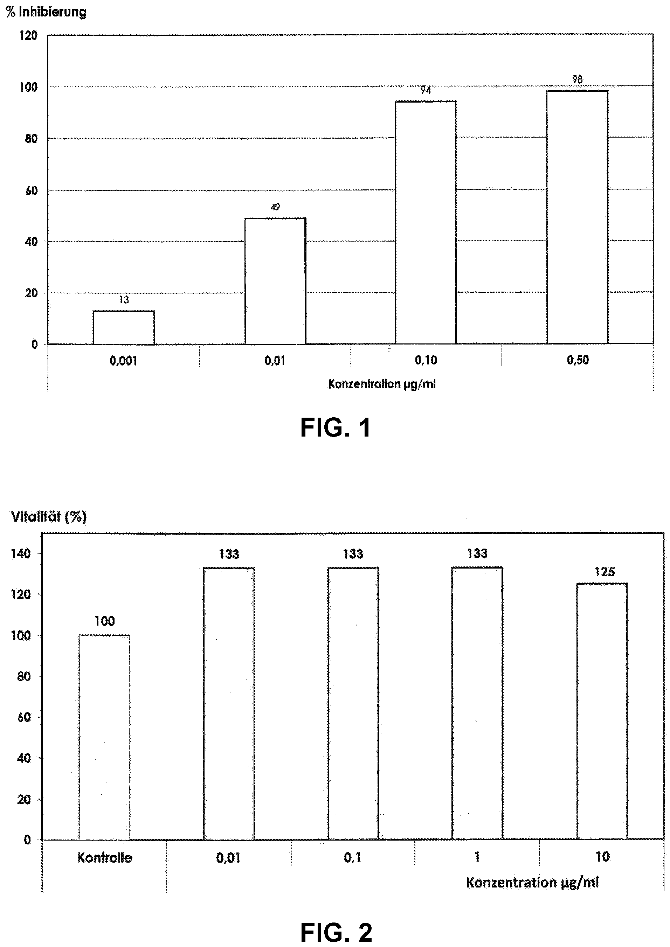

- Fig. 1: die konzentrationsabhängige Tyrosinasehemmung durch ein die Extrakte aus Rosenwurz (*Rhodiola rosea*) : Knotentang (*Ascophyllum nodosum*) : Purpur-Rotalge (*Porphyridium cruentum*) im Verhältnis von 2:2:1 aufweisenden Kosmetikums;
- Fig. 2: den konzentrationsabhängigen Einfluss des die Extrakte aus Rosenwurz (*Rhodiola rosea*) : Knotentang *(Ascophyllum nodosum*) : Purpur-Rotalge (*Porphyridium cruentum*) im Verhältnis von 2:2:1 aufweisenden Kosmetikums auf die Vitalität von normalen menschlichen dermalen Fibroblasten (*normal human dermal fibroblasts*; NHDF);

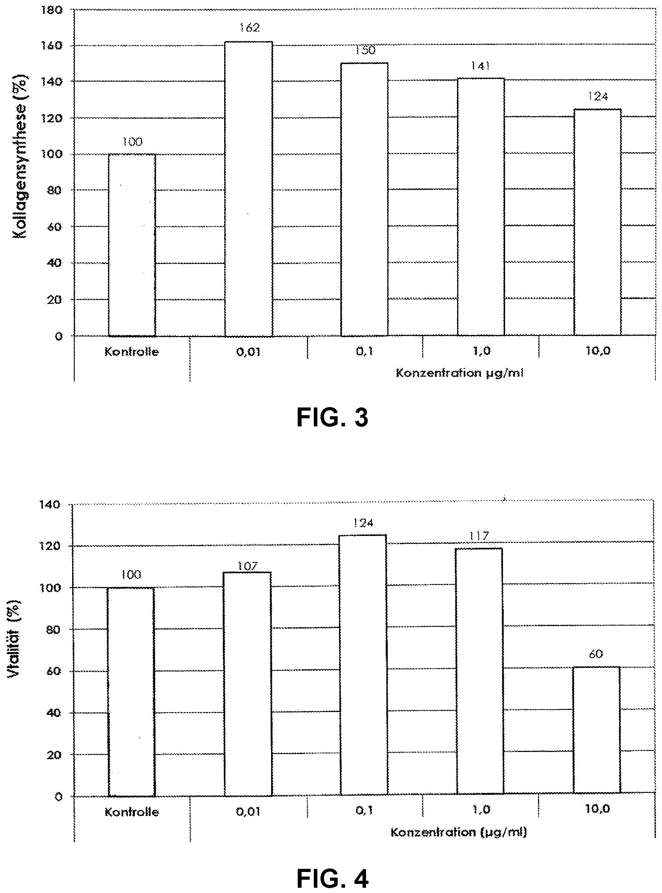

- Fig. 3: den konzentrationsabhängigen Einfluss des die Extrakte aus Rosenwurz (*Rhodiola rosea*) : Knotentang (*Ascophyllum nodosum*) : Purpur-Rotalge (*Porphyridium cruentum*) im Verhältnis von 2:2:1 aufweisenden Kosmetikums auf die Kollagensynthese von normalen menschlichen dermalen Fibroblasten (NHDF);
- Fig. 4: die zellprotektive Wirkung des die Extrakte aus Rosenwurz (*Rhodiola rosea*) : Knotentang (*Ascophyllum nodosum*): Purpur-Rotalge (*Porphyridium cruentum*) im Verhältnis von 2:2:1 aufweisenden Kosmetikums bei normalen menschlichen dermalen Fibroblasten (NHDF) bei Bestrahlung mit nahem UV-Licht mit 20 J/cm²; und

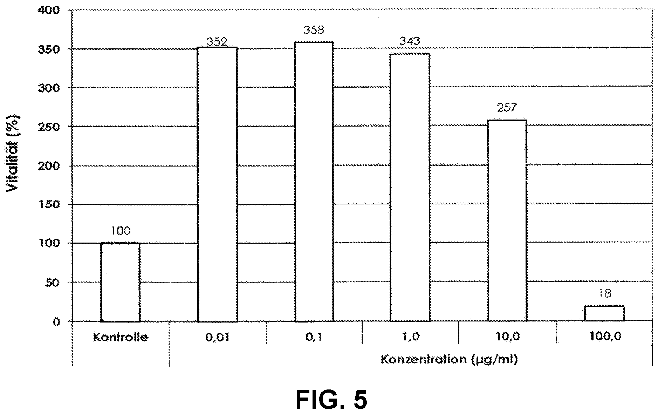

- Fig. 5: die zellgenerative Wirkung des die Extrakte aus Rosenwurz (*Rhodiola rosea*): Knotentang (*Ascophyllum nodosum*) : Purpur-Rotalge (*Porphyridium cruentum*) im Verhältnis von 2:2:1 aufweisenden Kosmetikums nach Bestrahlung unbehandelter normaler menschlicher dermaler Fibroblasten (NHDF) mit nahem UV-Licht mit 20 J/cm².

Fig. 1 zeigt die konzentrationsabhängige Tyrosinasehemmung durch ein die Extrakte aus Rosenwurz (*Rhodiola rosea*): Knotentang (*Ascophyllum nodosum*): Purpur-Rotalge (*Porphyridium cruentum*) im Verhältnis von 2:2:1 aufweisenden Kosmetikums.

Der Einfluss des Kosmetikums auf die Tyrosinaseaktivität wurde anhand des Dopachrom-Assays gernessen. L-Dopa reagiert wird bei Anwesenheit von Sauerstoff zum farbigen Dopchrom umgesetzt, das bei einer Wellenlänge von λ=475 nm erfasst wurde.

Fig. 1 zeigt, dass das Enzym Tyrosinase durch das erfindungsgemäße Kosmetikum konzentrationsabhängig inhibiert wird. Jede Messung erfolgte zweifach, in parallelen Ansätzen. Bereits eine Konzentration von 0,01 µg/ml führen zu einer 50 %-igen Inhibierung der Enzymaktivität, wobei die maximale Inhibierung bereits bei einer Konzentration von 0,1 µg/ml erreicht wird.

Mit steigender Tyosinaseinhibierung geht eine Rücknahme der Melaninproduktion und damit die angestrebte Hautaufhellung einher.

Fig. 2 zeigt den konzentrationsabhängigen Einfluss des die Extrakte aus Rosenwurz (*Rhodiola rosea*): Knotentang (*Ascophyllum nodosum)* : Purpur-Rotalge (*Porphyridium cruentum*) im Verhältnis von 2:2:1 aufweisenden Kosmetikums auf die Vitalität von normalen menschlichen dermalen Fibroblasten (*normal human dermal fibroblasts*; NHDF).

Die Wirkung des beanspruchten Kosmetikums auf die Stoffwechselaktivität wvon Hautzellen wurde mit dem MTT-Test an primären humanen Fibroblasten (*normal human dermal fibroblasts*; NHDF-Zellen) evaluiert. Dieser Test basiert auf der von Mosmann beschriebenen Methode zur Messung der Zell-Proliferation und Vitalität (Mosmann T. Rapid colorimetric assay for cellular growth and survival: application to proliferation and cytotoxicty assays. J Immunol Methods 16, 55 (1983)).

Diese Untersuchung ergab, dass die Vitalität der NHDF-Zellen im Konzentrationsbereich von 0,01 µg/ml bis 10 µg/ml stimuliert wird. Die maximale Stimulation wurde bereits bei einer Konzentration von 0,01 µg/ml erreicht.

Fig. 3 zeigt den konzentrationsabhängigen Einfluss des die Extrakte aus Rosenwurz (*Rhodiola rosea*) : Knotentang *(Ascophyllum nodosum)* : Purpur-Rotalge (*Porphyridium cruentum*) im Verhältnis von 2:2:1 aufweisenden Kosmetikums auf die Kollagensynthese von normalen menschlichen dermalen Fibroblasten (NHDF).

Mit Hilfe des Sircol Collagen Assays (Bicolor Ltd., Belfast) wurde der Einfluss des erfindungsgemäßen Kosmetikums auf die Kollagenbildung von normalen humanen dermalen Fibroblasten untersucht. Diese Untersuchungen zeigen, dass die Applikation des Kosmetikums bei einer Konzentration von 0,01 µg/ml eine Steigerung der Kollagensynthese um 62 % bewirken konnte - dieser Effekt deutet auf eine verbesserte Vitalität der Fibroblastenzellen hin.

Desweiteren konnten *in vitro* Tests an mit nahem UVLicht (UV-A: 380nm - 315 nm) bestrahlten NHDF-Zellen zeigen, dass das Kosmetikum nach der Erfindung Schädigungen der Haut durch UV-Strahlung sowohl vor als auch nach Applikation reduzieren kann. Das Kosmetikum ist also in der Lage, vor toxischen Effekten der UV-Strahlen zu schützen, als auch in den Zellen aufgetretene Schäden zu reduzieren.

Fig. 4 zeigt die zellprotektive Wirkung des die Extrakte aus Rosenwurz (*Rhodiola rosea*): Knotentang (*Ascophyllum nodosum*): Purpur-Rotalge (*Porphyridium cruentum*) im Verhältnis von 2:2:1 aufweisenden Kosmetikums bei normalen menschlichen dermalen Fibroblasten (NHDF) bei Bestrahlung mit nahem UV-Licht mit 20 J/cm².

Bei Applikation des Kosmetikums in einer Konzentration von 0,1 µg/ml vor der Bestrahlung mit nahem UV-Licht mit 20 J/cm² ergab sich eine 24 % höhere Vitalität bestrahlter und behandelter NHDF-Zellen gegenüber NHDF-Zellen, die bestrahlt und unbehandelt waren (*"Kontrolle"*).

Fig. 5 zeigt schließlich die zellgenerative Wirkung des die Extrakte aus Rosenwurz (*Rhodiola rosea*): Knotentang (*Ascophyllum nodosum*): Purpur-Rotalge (*Porphyridium cruentum*) im Verhältnis von 2:2:1 aufweisenden Kosmetikums nach Bestrahlung unbehandelter normaler menschlicher dermaler Fibroblasten (NHDF) mit nahem UV-Licht mit 20 J/cm²,

Bei Applikation des Kosmetikums in einer Konzentration von bereits wenigstens 0,01 µg/ml nach der Bestrahlung mit nahem UV-Licht mit 20 J/cm² ergab sich eine 2-fach höhere Vitalität bestrahlter und nach Bestrahlung behandelter NHDF-Zellen gegenüber NHDF-Zellen, die bestrahlt und anschließend unbehandelt geblieben sind (*"Kontrolle"*)*.*

Neben der Hautaufhellung und den protektiven wie auch regenerativen Eigenschaften, weist das Kosmetikum weitere vorteilhafte Eigenschaften auf, die auch im Bereich Anti-Aging eingesetzt werden können. So konnte eine Steigerung der Hautfeuchtigkeit um 60 %, eine Faltenreduzierung um 19 %, eine Verbesserung der Hautelastizität um 10 %, eine Verbesserung der Hautglättung um 16 % und eine Verbesserung der Hautfestigkeit um 19 % festgestellt werden.

Darüber hinaus ist das Kosmetikum gut hautverträglich.

## Patentansprüche

1. Hautaufhellendes Kosmetikum mit einem die Melanogenese hemmenden Wirkstoff,
**gekennzeichnet durch**
einen Extrakt aus dem Knotentang (*Ascophyllum nodosum*) und einen Extrakt aus der Purpur-Rotalge (*Porphyridium cruentum*), wobei der die Melanogenese hemmende Wirkstoff ein Extrakt aus Rosenwurz (*Rhodiola rosea*) ist.

2. Kosmetikum nach Anspruch 1, **dadurch gekennzeichnet, dass** der Extrakt aus dem Knotentang (*Ascophyllum nodosum*) CAS-Nr. 84775-78-0 und/oder der Extrakt aus der Purpur-Rotalge (*Porphyridium cruentum*) CAS-Nr. 223751-77-7 ist.

3. Kosmetikum nach einem der vorhergehenden Ansprüche, **dadurch gekennzeichnet, dass** der Extrakt aus Rosenwurz (*Rhodiola rosea*) CAS-Nr. 97404-52-9 ist.

4. Kosmetikum nach Anspruch 1, **gekennzeichnet durch** ein Gewichtsverhältnis der Extrakte aus Rosenwurz : Knotentang: Purpur-Rotalge von 2:2:1.

5. Kosmetikum nach einem der vorhergehenden Ansprüche, **dadurch gekennzeichnet, dass** das Kosmetikum eine die Vitalität von Fibroblasten stimulierende Wirkung aufweist.

6. Kosmetikum nach einem der vorhergehenden Ansprüche, **dadurch gekennzeichnet, dass** das Kosmetikum eine die Kollagensynthese steigernde Wirkung aufweist.

7. Kosmetikum nach einem der vorhergehenden Ansprüche, **dadurch gekennzeichnet, dass** das Kosmetikum eine zellprotektive Wirkung vor dem schädigenden Einfluss von UV-Strahlung aufweist.

8. Kosmetikum nach einem der vorhergehenden Ansprüche, **dadurch gekennzeichnet, dass** das Kosmetikum eine zellgenerative Wirkung nach UV-Bestrahlung aufweist.

## Claims

1. Skin-lightening cosmetic comprising an active ingredient which inhibits melanogenesis, **characterized by** an extract from rockweed (*Ascophyllum nodosum*) and an extract from purple-red alga (*Porphyridium cruentum*), the active ingredient which inhibits melanogenesis being an extract from rose root (*Rhodiola* rosea).

2. Cosmetic according to claim 1, **characterized in that** the extract from rockweed (*Ascophyllum nodosum*) is CAS no. 84775-78-0 and/or the extract from purple-red alga (*Porphyridium cruentum*) is CAS no. 223751-77-7.

3. Cosmetic according to either of the preceding claims, **characterized in that** the extract from rose root (*Rhodiola rosea*) is CAS no. 97404-52-9.

4. Cosmetic according to claim 1, **characterized by** a weight ratio of the extracts from rose root: rockweed : purple-red alga of 2:2:1.

5. Cosmetic according to any of the preceding claims, **characterized in that** the cosmetic has an effect that stimulates the vitality of fibroblasts.

6. Cosmetic according to any of the preceding claims, **characterized in that** the cosmetic has an effect that increases collagen synthesis.

7. Cosmetic according to any of the preceding claims, **characterized in that** the cosmetic has a cell-protective effect against the damaging influence of UV radiation.

8. Cosmetic according to any of the preceding claims, **characterized in that** the cosmetic has a cell-generating effect after UV irradiation.

## Revendications

1. Produit cosmétique éclaircissant la peau, comportant une substance active inhibant la mélanogenèse, **caractérisé par** un extrait de goémon noir (*Ascophyllum nodosum*) et un extrait d'algue rouge pourpre (*Porphyridium cruentum*), la substance active inhibant la mélanogénèse étant un extrait de racine de rose (*Rhodiola* rosea).

2. Produit cosmétique selon la revendication 1, **caractérisé en ce que** l'extrait de goémon noir (*Ascophyllum nodosum*) est le n° CAS 84775-78-0 et/ou **en ce que** l'extrait d'algue rouge pourpre (*Porphyridium cruentum*) est le n° CAS 223751-77-7.

3. Produit cosmétique selon l'une des revendications précédentes, **caractérisé en ce que** l'extrait de racine de rose (*Rhodiola rosea*) est le n° CAS 97404-52-9.

4. Produit cosmétique selon la revendication 1, **caractérisé par** un rapport pondéral des extraits de racine de rose : goémon noir : algue rouge pourpre de 2:2:1.

5. Produit cosmétique selon l'une des revendications précédentes, **caractérisé en ce que** le produit cosmétique présente un effet stimulant la vitalité des fibroblastes.

6. Produit cosmétique selon l'une des revendications précédentes, **caractérisé en ce que** le produit cosmétique présente un effet augmentant la synthèse du collagène.

7. Produit cosmétique selon l'une des revendications précédentes, **caractérisé en ce que** le produit cosmétique présente un effet de protection des cellules contre l'influence néfaste du rayonnement UV.

8. Produit cosmétique selon l'une des revendications précédentes, **caractérisé en ce que** le produit cosmétique présente un effet générateur de cellules après irradiation UV.
